# EUROPEAN PATENT SPECIFICATION

(11) **EP 3 123 977 B1**
(45) Date of publication and mention of the grant of the patent: **01.05.2019**
(21) Application number: 15770266.3
(22) Date of filing: 25.03.2015
(51) Int. Cl.: A61C 15/02, A61Q 11/00, A46B 3/00, A61K 8/37, A61K 8/73

(54) **INTERDENTAL CLEANING TOOL AND METHOD FOR MANUFACTURING THEREOF**
INTERDENTALES REINIGUNGSINSTRUMENT UND VERFAHREN ZUR DESSEN HERSTELLUNG
OUTIL DE NETTOYAGE INTERDENTAIRE ET SON PROCÉDÉ DE FABRICATION

(30) Priority: 27.03.2014 JP 2014066723
(43) Date of publication of application: 01.02.2017
(73) Proprietor: Sunstar Suisse SA, 1163 Etoy (CH)
(72) Inventor: KATO Keisuke, Takatsuki-shi Osaka 569-1195 (JP); OSHIMA Yoshiyuki, Takatsuki-shi Osaka 569-1195 (JP)
(74) Representative: Vossius & Partner Patentanwälte Rechtsanwälte mbB
(86) International application number: PCT/JP2015/059185
(87) International publication number: WO 2015/147076

(56) References cited:
- WO-A1-2013/176297
- WO-A1-2013/176297
- WO-A1-2014/023424
- JP-A- H 105 251
- JP-A- 2000 342 605
- JP-A- 2000 342 605
- JP-A- 2008 156 288
- US-A- 6 158 444

## Description

### Technical Filed

The present invention relates to an interdental cleaning tool to which a perfume is applied, and a method for manufacturing the same.

### Background Art

When an oral cavity is cleaned in daily life, an interdental cleaning tool has hitherto been used in order to clean plaque (dental plaque) between teeth, which cannot be cleaned by using a usual toothbrush.

In prior interdental cleaning tools, a tool containing a handle base and a cleaning part in which a nylon brush is fixed on a metal wire has been mainly used, but a case where the gums are injured or a pain is felt has often been seen when it is used. An interdental cleaning tool whose cleaning part is formed of a synthetic resin, having a soft comfort level, is proposed.

In recent years, an interdental cleaning tool in which a perfume is applied to a synthetic resin cleaning part by some method, whereby, for example, cool sensation is improved when it is used, has been proposed, as an interdental cleaning tool having a further additional value.

As such interdental cleaning tool in which the perfume is applied to the cleaning part, Patent Document 1 proposes an interdental cleaning tool in which a cleaning part is covered with a covering film containing a powdery aroma component. When the powdery aroma component is used, however, a coatability of the covering film on the cleaning part and a cool sensation caused by the aroma component are easily influenced by a concentration or a particle size of the powdery aroma component, and there is a problem in which it is difficult to maintain uniformity of products.

On the other hand, Patent Document 2 does not relate to an invention of an interdental cleaning tool, but proposes an invention concerning a toothbrush having a synthetic resin oral cavity cleaning part, in which a liquid perfume is kneaded into the synthetic resin oral cavity cleaning part. When the perfume is kneaded into the synthetic resin as above, however, strong smell is emitted from the perfume in the production steps, and thus, in order to prevent worsening of working environments in the production line, a deodorization step is necessary during the production, which cause poor workability and cost rise. In addition, the perfume quickly volatilizes after the productization, and it is difficult to hold the perfume in the cleaning part for a long period of time. Further, there is also a problem in which when the perfume is held in the cleaning part as above, the scent can be felt when it is smelled, but the scent can hardly be felt when it is used in the oral cavity, and thus the taste or cool sensation derived from the perfume can be insufficiently obtained when it is used, which is the essential part.

Patent Document 3 discloses an interdental cleaning tool according to the preamble of claim 1 and a method for manufacturing said interdental cleaning tool.

### Citation List

### Patent Literature

Patent Document 1: JP-ANo. 2008-11944
Patent Document 2: JP-T No. 2009-540990
Patent Document 3: WO 2013/176297

### Summary of Invention

### Technical Problem

In view of the circumstance as described above, the present invention aims at, in an interdental cleaning tool having a cleaning part in which the surface of a cleaning body is covered with a perfume layer, providing an interdental cleaning tool in which a larger amount of a flavor component such as an oil-soluble perfume is applied on the surface of an elastomeric cleaning body having high water-repellency and is left thereon, a soft sense of use as the interdental cleaning tool is provided, flavor caused by the flavor component can be effectively felt when the interdental cleaning tool is used, and the flavor component can be maintained for a long period of time. Solution to Problem

As a result of repeating painstaking studies, the present inventors have found that, in an interdental cleaning tool containing a handle base, and a cleaning body which is substantially formed of a polystyrene elastomer and whose surface is covered with a perfume layer, when the perfume layer is formed to contain a glycerin fatty acid ester, an oil-soluble perfume, and gum arabic and/or starch octenylsuccinate as a water-soluble covering base, it is possible to solve the problems described above.

In addition, the present inventors have also found that such an interdental cleaning tool can be manufactured in a method containing a cleaning body-forming step in which a cleaning body having a cleaning part is formed at an end portion of a handle base; a coating step in which a cleaning part surface of the cleaning body is coated with a treatment liquid in which a flavor component and the like are stably emulsified (hereinafter referred simply to as an "emulsion") to form a perfume layer; and a drying step in which the solvent of the emulsion coated is vaporized and removed, and it is possible to solve the problems described above.

Thus, the present invention relates to:
[1] An interdental cleaning tool containing: a handle base formed of a synthetic resin; and a cleaning part disposed on an end portion of the handle base, wherein a surface of a cleaning body substantially formed of a polystyrene elastomer in the cleaning part is covered with a perfume layer, and the perfume layer contains a glycerine fatty acid ester, an oil-soluble perfume as a flavor component, and gum arabic and/or starch octenylsuccinate as a water-soluble covering base;
[2] The interdental cleaning tool according to [1] above, wherein the polystyrene elastomer has a Shore hardness A of 30 to 60;
[3] The interdental cleaning tool according to [1] or [2] above, wherein the oil-soluble perfume contains at least one member selected from menthol and menthol analogues;
[4] The interdental cleaning tool according to any of [1] to [3] above, wherein the perfume layer contains the gum arabic and/or the starch octenylsuccinate in a content of 40% by weight or more and 70% by weight or less;
[5] The interdental cleaning tool according to any of [1] to [4] above, wherein the cleaning body is formed so as to cover a surface of the end portion of the handle base;
[6] The interdental cleaning tool according to any of [1] to [5] above, wherein the cleaning body contains a protrusion radially formed from the handle base;
[7] A method for manufacturing an interdental cleaning tool containing a handle base formed of a synthetic resin, and a cleaning part disposed on an end portion of the handle base, containing: a cleaning body-forming step in which a cleaning body substantially formed of a polystyrene elastomer is disposed on the end portion of the handle base; a coating step in which a surface of the cleaning body is coated with an emulsion containing a glycerine fatty acid ester, an oil-soluble perfume as a flavor component, gum arabic and/or starch octenylsuccinate as a water-soluble covering base, and water as a solvent; and a drying step in which the solvent is removed from the emulsion coated on the surface of the cleaning body;
[8] The method for manufacturing an interdental cleaning tool according to [7] above, wherein the emulsion contains the gum arabic and/or the starch octenylsuccinate in a content of 5% by weight or more and 30% by weight or less;
[9] The method for manufacturing an interdental cleaning tool according to [7] or [8] above, wherein, in the coating step, the surface of the cleaning body is spray-coated with the emulsion; and
[10] The method for manufacturing an interdental cleaning tool according to any of [7] to [9] above, wherein the water in the solvent is contained in a content of 40% by weight or more.

The present invention relates to an interdental cleaning tool containing a handle base formed of a synthetic resin, and a cleaning part disposed at an end portion of the handle base, wherein the cleaning part is formed of a cleaning body substantially formed of a polystyrene elastomer, and a perfume layer covering a surface of the cleaning body, and the perfume layer contains glycerin fatty acid ester, an oil-soluble perfume as a flavor component, and gum arabic and/or starch octenylsuccinate as a water-soluble covering base.

The oil-soluble perfume may contain at least one compound selected from monocyclic terpene compounds, peppermint (oil), spearmint (oil), and Japanese mint (oil).

The present invention relates also to a method for manufacturing an interdental cleaning tool containing a handle base formed of a synthetic resin and a cleaning part disposed at an end portion of the handle base, containing:
a cleaning body-forming step in which a cleaning body substantially formed of a polystyrene elastomer is disposed at an end portion of the handle base, a coating step in which an emulsion containing a glycerin fatty acid ester, an oil-soluble perfume as a flavor component, gum arabic and/or starch octenylsuccinate as a water-soluble covering base, and water as a solvent, is applied to a surface of the cleaning body, and a drying step in which the solvent is removed from the emulsion applied to the surface of the cleaning body.

### Advantageous Effects of Invention

According to the interdental cleaning tool and the manufacturing method therefor according to the present invention described above, an interdental cleaning tool can be provided, in the interdental cleaning tool in which the flavor component is applied to the surface of the cleaning body, which interdental cleaning tool can maintain the quality by maintaining the flavor component for a long period of time while the soft sense of use as the interdental cleaning tool is provided and it is possible to effectively feel the flavor by the flavor component when it is used.

### Brief Description of Drawings

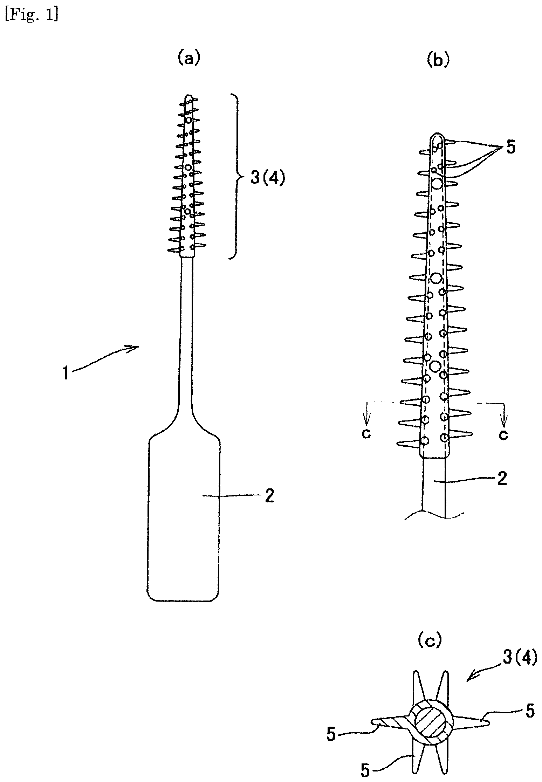

Fig. 1(a) is one example of embodiments of an interdental cleaning tool according to the present invention.
Fig. 1(b) is an enlarged view of a cleaning part in an embodiment of an interdental cleaning tool according to the present invention.
Fig. 1(c) is a c-c cross-sectional view of a cleaning part in an embodiment according to an interdental cleaning tool according to the present invention.

### Description of Embodiments

An interdental cleaning tool according to the present invention comprises a handle base, and a cleaning part provided at an end portion of the handle base. The cleaning part comprises a cleaning body substantially formed of a polystyrene elastomer, and a perfume surface layer which covers the cleaning body. Further, the perfume layer contains a glycerin fatty acid ester, an oil-soluble perfume as a flavor component, and gum arabic and/or starch octenylsuccinate as a water-soluble covering base.

Embodiments of the interdental cleaning tool according to the present invention are explained below, referring to drawings.

An interdental cleaning tool 1 according to the present invention comprises, as shown in Fig. 1 (a), a handle base 2 formed of a synthetic resin, and a cleaning part 3 provided at an end portion of the handle base 2. The synthetic resin used for the handle base 2 is not particularly limited, and known synthetic resins used for a handle of an interdental cleaning tool can be widely used. Specifically, thermoplastic synthetic resin materials can be employed such as polypropylene, polyethylene, polyethylene terephthalate, polycyclohexylenedimethylene terephthalate, saturated polyester resins, poly(methyl methacrylate), cellulose propionate, polyurethane, polyamide, polycarbonate, and ABS (acrylonitrile-butadiene-styrene). It is preferable to add, as an additive, a fiber material such as glass fiver, carbon fiber or aramid fiber or a mineral such as mica or talc to the synthetic resin material forming the handle base.

The cleaning part 3 is formed of a cleaning body 4 and a perfume layer which covers the body. The cleaning body 4 is substantially formed of a polystyrene elastomer. The term "substantially" herein means that the part may contain an elastomer other than the polystyrene elastomer or another additive, but a main forming material of the cleaning body 4 is the polystyrene elastomer, and the part may contain, of course, the polystyrene elastomer alone. A Shore A hardness of the polystyrene elastomer is not particularly limited, and it is preferable to adjust it to 30 to 60. According to the method for manufacturing an interdental cleaning tool of the present invention, described below, when the body has the structure described above, an effect that an effect of the flavor component can be maintained for a long period of time can be obtained. In addition, it is preferable to adjust the Shore A hardness of the polystyrene elastomer from 30 to 40, in order to obtain the soft sense of use.

Hitherto, the cleaning part of the interdental cleaning tool has been often formed by putting a nylon brush between metal wires. In the cleaning body 4 in the present invention, however, it is desirable to form the end portion of the handle base 2 so as to be covered, in order to reduce a user's fear to a metal or damage to gum. When the cleaning body 4 is formed so as to have projections 5 radially formed from the handle base 2 in a circumferential direction (see particularly Fig. 1 (c)) or wall plate-like projections formed in a circumferential direction, preferably, not only the cleaning property can be improved but also a large amount of the perfume layer can be applied due to the increased surface area and the improved residual property of the applied liquid between fine structures close to each other.

The perfume layer contains a glycerin fatty acid ester, an oil-soluble perfume as a flavor component, and gum arabic and/or starch octenylsuccinate as a water-soluble covering base.

As the glycerin fatty acid ester, it is preferable to use oil-soluble esters and, for example, triglyceride of a glycerin fatty acid ester can be used, but it is not limited thereto. The glycerin fatty acid ester functions as a dispersing agent of the oil-soluble perfume as well as functions as a solubilizer, and it dissolves a part of the oil-soluble perfumes. In particular, when an emulsion is used when the perfume layer is formed, it is necessary that the flavor component is made exist in the emulsion as oil-soluble liquid. For that reason, it is preferable to contain the glycerin fatty acid ester in an amount of 0.1 to 30% by weight, more preferably 0.1 to 4% by weight relative to the total weight of the perfume layer formed. The term "total weight of the perfume layer" herein refers to a total amount of the perfume layer covering the cleaning body 4, and, for example, when an emulsion is used for forming the perfume layer, the total weight of the perfume layer can be calculated as a weight obtained by subtracting a weight of a solvent from a weight of an emulsion.

The term "oil-soluble perfume" in the present invention means a flavoring composition such as a flavoring compound or an essential oil having an aqueous solubility of less than 1 g/100 mL at 20°C. The term "flavoring property" herein means a property of its smell being smelled at 20°C.

As the oil-soluble perfume, known oil-soluble perfumes used for food can be appropriately used. For example, terpene or terpenoid is preferable for providing a cool sensation. Specifically, the perfume may include menthol or analogs thereof, and natural perfumes containing a large amount of menthol, i.e., menthol, menthyl lactate, menthyl acetate, monomenthyl succinate, menthyl gluconate, menthoxyacetic acid, isopulegol, spearmint (oil), Japanese mint (oil), and the like. The content of the oil-soluble perfume is not particularly limited so long as a desired smell can be felt when it is used, and the content is preferably from 10 to 60% by weight relative to the perfume layer.

The other oil-soluble perfumes may be used, but is not limited to, such as natural perfumes including peppermint oil, spearmint oil, Japanese mint oil, anise oil, eucalyptus oil, wintergreen oil, cassia oil, clove oil, thyme oil, sage oil, lemon oil, orange oil, peppermint oil, cardamom oil, coriander oil, mandarine oil, lime oil, lavender oil, rosemary oil, laurel oil, chamomile oil, caraway oil, marjoram oil, bay oil, lemongrass oil, orignum oil, pineneedle oil, neroli oil, rose oil, jasmine oil, iris concrete, absolute peppermint, absolute rose, orange flower, and the like; treated (front reservoir part cut, rear reservoir part cut, fractionally distilled, liquid extracted, extracted, powdered, or the like) perfumes of the natural perfumes; single item perfumes including menthol, carvone, anethole, cineole, methyl salicylate, cinnamic aldehyde, eugenol, thymol, linalol, linalyl acetate, limonene, menthone, menthyl acetate, pinene, octyl aldehyde, citral, pulegone, carvyl acetate, anise aldehyde, cumin aldehyde, carvacrol, cymene, hinokitiol, hexa-p-cymene, isopulegol, menthyl lactate, menthoxyacetic acid, neomenthol, perillaldehyde, phellandrene, piperitone, sobrerol, terpinene, terpineol, menthene, sylvestrene, perillaldehyde, menthyl acetate, menthyl lactate, menthofuran, terpinolene, ethyl vanillin, benzaldehyde, camphor, borneol, fenchone, trimethyl glycine menthyl ester, ethyl acetate, ethyl butyrate, allyl cyclohexane propionate, methyl anthranilate, ethyl methyl phenyl glycidate, vanillin, undecalactone, hexanal, ethyl alcohol, propyl alcohol, butanol, isoamyl alcohol, hexanol, dimethyl sulfide, cyclotene, furfural, trimethylpyrazine, ethyl lactate, ethyl thioacetate, and the like; and further strawberry flavor, apple flavor, banana flavor, pineapple flavor, grape flavor, mango flavor, butter flavor, milk flavor, fruit mix flavor, tropical fruit flavor, and the like. Of these, monocyclic terpene compounds such as isopulegol, ethyl vanillin, eugenol, carvone, carvacrol, cuminaldehyde, menthyl acetate, sylvestrene, cymene, cinnamic aldehyde, sobrerol, thymol, terpinene, terpineol, terpinolene, neomenthol, vanillin, hinokitiol, pireritone, hexahydro-p-cymene, benzaldehyde, perillaldehyde, phellandrene, pulegone, menthyl acetate, menthyl lactate, menthoxyacetic acid, menthone, menthol, menthane, menthene, and limonene, and peppermint (oil), spearmint (oil), and Japanese mint (oil) are preferable; monocyclic monoterpene compounds such as isopulegol, carvone, carvacrol, menthyl acetate, terpinene, terpineol, piperitone, perillaldehyde, phellandrene, pulegone, menthyl acetate, menthyl lactate, menthoxyacetic acid, menthol, and limonene, peppermint (oil), spearmint (oil), Japanese mint (oil) are more preferable; and monocyclic monoterpene compounds such as menthyl acetate, menthyl lactate, menthoxyacetic acid, and menthol, and peppermint (oil), spearmint (oil), and Japanese mint (oil) are still more preferable.

An embodiment in which an oil-soluble perfume capable of providing a cool sensation as a flavor, such as menthol or a menthol analog is used together with a cool sensation-enhancing agent is preferable, because refreshingness is improved when it is used. The cool sensation-enhancing agent may include quinic acid, chlorogenic acid, caffeoylquinic acid, dicaffeoylquinic acid, 2-methyl maleic acid monomenthyl glutaric acid ester, menthyl 3-hydroxybutyrate, 3-methoxy-1,2-propanediol, N-ethyl-p-menthane-3-carboxamide, N- {(ethoxycarbonyl)methyl} -p -menthane - 3 -carboxamide, N,2,3-trimethyl-2-(1-methylethyl)butanamide, N-para-benzene acetonitrile menthanecarboxamide, menthoneglycerol ketal, methyl lactate, hydroxymethyltrimethylcyclohexanol, menthyl-9-hydroxynonylcarbonate, mentholethyleneglycol carbonate, mentholpropyleneglycol carbonate, and the like.

In the present invention, as the water-soluble covering base, gum arabic and/or starch octenylsuccinate are/is used. When it is used, it is possible to stably form an uniform covering film containing the flavor component (the covering film containing the flavor component is hereinafter referred to as a "perfume layer") on the surface of the cleaning body formed of the elastomer generally having the water-repellency, and the oil-soluble perfume such as menthol or menthol analog, which is inherently volatilized within a short time, can be maintained in the perfume layer for a long period of time. In addition, the gum arabic and/or the starch octenylsuccinate form a flexible covering film thereby providing the perfume layer with the flexibility, and thus the perfume layer is less likely to be peeled off from the cleaning body when the interdental cleaning tool is used. It is considered that, by this way, more flavor component than necessary does not flow from the perfume layer in a short time when the interdental cleaning tool is used, and the flavor component is gradually eluted as the water-soluble covering base is gradually eluted by saliva, and thus the cool sensation can be maintained for a long period of time during the use. Furthermore, as described below, when an emulsion is used when the perfume layer is formed, the emulsification stability of the emulsion can be improved, because it is considered that the gum arabic and/or the starch octenylsuccinate has a surfactant effect or a dispersion stabilizing effect to disperse the flavor component such as the oil-soluble perfume in water, which is a continuous layer.

The content of the gum arabic and/or the starch octenylsuccinate in the perfume layer is not particularly limited, and in order to uniformly form the perfume layer on the surface of the cleaning body, and to effectively hold the flavor component such as the oil-soluble perfume in the perfume layer, it is preferable to contain the gum arabic and/or the starch octenylsuccinate in a content within a range of 40% by weight or more and 70% by weight or less relative to the total weight of the perfume layer. The term "total weight of the perfume layer" herein refers to the total weight of the perfume layer covering the cleaning body 4, and, for example, when an emulsion is used for forming the perfume layer, the total weight of the perfume layer can be calculated as a weight obtained by subtracting a weight of a solvent from a weight of an emulsion.

In the present invention, the emulsification stability of the emulsion can be improved by using, as the water-soluble covering base, the gum arabic and/or the starch octenylsuccinate, when the emulsion is used for forming the perfume layer, as described above. However, according to the study by the present inventors, it has been found that if the gum arabic and/or the starch octenylsuccinate is used together with polyoxyethylene hardened castor oil, polyglycerin fatty acid ester, sorbitan fatty acid ester (polysorbate) or sucrose fatty acid ester, which are surfactants, the emulsification stability may sometimes be reduced. The polyoxyethylene hardened castor oil, the polyglycerin fatty acid ester, the sorbitan fatty acid ester, or the sucrose fatty acid ester is contained in a content of preferably 30% by weight or less, more preferably 10% by weight or less relative to the total amount of the perfume layer finally formed, and it is particularly preferable that such component is not substantially contained (0% by weight).

The perfume layer may contain, if necessary, other components such as a pH-controlling agent, a preservative, and a sweetener to control flavor. The contents of the other components may be appropriately decided within a range in which the effects thereof can be exhibited without impairing the effects of the present invention.

As the pH-controlling agent, known pH-controlling agents can be used. It is possible to use specifically citric acid, gluconic acid, succinic acid, potassium carbonate, sodium hydrogencarbonate, lactic acid, and the like, but it is not limited thereto.

As the preservative, known preservatives can be used. It is possible to use specifically benzoic acid salts such as sodium bonzoate, paraoxybenzoic acid esters such as methyl paraben and ethyl paraben, sorbic acid, potassium sorbate, propylene glycol, polylysine and the like, but the preservative is not limited thereto.

As the sweetener, known sweeteners can be used. It is possible to use specifically, glycose, fructose, sucrose, erythritol, trehalose, maltitol, palatinose, xylitol, sorbitol, and the like. In addition to these compounds, it is possible to use so-called high rank sweeteners, such as aspartame, steviol glycoside, advantame, neotame, saccharin, sucralose, acesulfame potassium, and the like, but it is not, of course, limited thereto.

A preferable embodiment of the interdental cleaning tool according to the present invention is an interdental cleaning tool comprising a handle base formed of a synthetic resin, and a cleaning body disposed so that it covers a circumference of the handle base at an end portion of the handle base, wherein the cleaning body is formed of a polystyrene elastomer having a Shore hardness A of substantially 30 to 60, more preferably 30 to 40, and the perfume layer is formed on a surface of the cleaning body. An embodiment in which the perfume layer covers uniformly the surface of the cleaning body is more preferable. In such embodiment, an interdental cleaning tool comprises a perfume layer preferably containing, as the flavor component, at least one compounds selected from menthol and menthol analogs, and 40% by weight or more and 70% by weight or less of the gum arabic and/or the starch octenylsuccinate, which are the water-soluble covering bases, and more preferably further containing the cool sensation-enhancing agent as the flavor component.

When the embodiments described above are adopted, the soft sense of use of the cleaning body can be secured. In addition, in such embodiments, the flavor component can be maintained for a long period of time in the cleaning body, or the cool sensation can be clearly made to be felt.

As another preferable embodiment of the interdental cleaning tool according to the present invention, for example, an interdental cleaning tool comprising a handle base formed of a synthetic resin, and a cleaning part disposed at an end portion of the handle base, wherein, in the cleaning part, a surface of a cleaning body substantially formed of a polystyrene elastomer having a Shore hardness A of 30 to 60, preferably 30 to 40 is covered with a perfume layer, and the perfume layer contains, as the flavor component, at least one oil-soluble perfume selected from menthol and menthol analogs and, as the water-soluble covering base, 40% by weight or more and 70% by weight or less of the gum arabic and/the starch octenylsuccinate, and 1% by weight or less of a component having a surfactant effect other than the gum arabic and/or the starch octenylsuccinate is a typically preferable embodiment. An embodiment in which as the flavor component, the cool sensation-enhancing agent is further contained in addition to the embodiment described above is more preferable.

In this case, while the menthol or the menthol analog, which has the excellent cool sensation but the poor cool sensation due to its high volatility, is used as the flavor component, it is possible to retain the flavor component in the perfume layer for a long period of time by using the gum arabic or the starch octenylsuccinate as described above. According to the method for manufacturing the interdental cleaning tool according to the present invention described below, because the polystyrene elastomer having a Shore hardness A of 30 to 60 is used, it is possible to apply a larger amount of an emulsion to the cleaning body in a coating step, and it is possible to provide the interdental cleaning tool in which the cool sensation is lasted for longer time.

Next, the method for manufacturing the interdental cleaning tool according to the present invention is explained. The manufacturing method according to the present invention comprises a cleaning body-forming step for forming the cleaning body at the end portion of the handle base, and a coating step and drying step for forming the perfume layer on the surface of the cleaning body.

The handle base can be produced by using a known synthetic resin used for the interdental cleaning tool, and widely employing a known method such as an injection molding.

Then, the cleaning body is provided at the end portion of the handle base. The cleaning body may be substantially formed of the polystyrene elastomer and widely employing a known method such as injection molding. More specifically, a method such as insert molding, a method in which the handle base is heat-melted or bonded using an adhesive to the cleaning body, which has been previously formed, and the like may be used, but the method is not, of course, limited thereto. In such methods, it is preferable to form the cleaning body so that the polystyrene elastomer has a Shore hardness A of 30 to 60. This can suppress liquid dripping caused when an emulsion for the perfume layer is coated in the coating step described below; that is, a sufficient amount of the emulsion is applied to and left on the surface of the cleaning body and thus the perfume layer having a sufficient thickness can be formed, and an effect to maintain the oil-soluble perfume in the perfume layer for a long period of time can be obtained.

After that, a coating step is performed in which an emulsion containing the flavor component, the water-soluble covering base, and a solvent such as water is coated on the surface of the cleaning body.

In the coating step, the emulsion containing the glycerin fatty acid ester, the oil-soluble perfume, the water-soluble covering base, the solvent, and the like is prepared, and the emulsion is coated on the surface of the cleaning body, whereby not only the cool sensation is improved but also the persistence of the cool sensation can be further expected, compared to a method in which the flavor component is kneaded with the synthetic resin as described in Patent Document 2. In addition, when the cleaning body is covered with the perfume layer, it is possible to uniformly leave a sufficient amount of the flavor component on the surface of the cleaning body by using the emulsion, compared to a case where a powdery flavor component is used, and thus not only the cool sensation is improved but also the occurrence of variation in the concentration of the flavor component can be suppressed, whereby the uniformity of the product quality can be improved. The coating method in the coating step is not particularly limited so long as the emulsion can be applied to the surface of the cleaning body, and may include, for example, a method in which the cleaning body is dipped in the emulsion, a method in which the emulsion is coated with a brush, a method in which the emulsion is sprayed, and the like. Of these, it is desirable to coat the emulsion by spraying. The spraying can uniformly coat the emulsion on the whole surface of the cleaning body, and the adhesive property of the emulsion can be improved.

The emulsion contains desirably the gum arabic and/or the starch octenylsuccinate in a content of 5% by weight or more and 30% by weight or less. In such case, the emulsification stability of the emulsion is improved and the perfume layer with desired properties can be formed, as well as the preferable spraying property can be obtained when the emulsion is spray-coated. In order to obtain the more preferable spraying property, it is preferable to adjust the viscosity of the emulsion to 200 mPa·s or less at 25°C. The adjustment of the viscosity of the emulsion varies depending on the kind and amount of the component contained in the emulsion, and the viscosity can be adjusted by controlling the amount of the gum arabic and/or the starch octenylsuccinate contained within a range in which the effects of the present invention are not impaired.

As described above, if the polyoxyethylene hardened castor oil, the polyglycerin fatty acid ester, the sorbitan fatty acid ester, and the sucrose fatty acid ester, which are surfactants, is contained in the emulsion, the emulsification stability of the emulsion may possibly be reduced. Accordingly, such component is preferably contained in a content of 10% by weight or less, more preferably 5% by weight or less relative to the emulsion, and it is particularly preferable that the component is not substantially contained, i.e., 0%.

Water is used as the solvent of the emulsion, in order to improve the emulsification, the solubilization and the dispersibility of oil. According to the study made by the present inventors, it has been found that when an organic solvent is used in addition to water as the solvent, the emulsification stability of the emulsion may sometimes be reduced. Such solvent may include, for example, water-soluble solvents, more specifically may include monovalent lower alcohols such as ethanol. The content of the organic solvent in the solvent of the emulsion is, accordingly, preferably 60% by weight or less, more preferably 10% by weight or less, and it is still more preferable that solvents other than the monovalent lower alcohol contained in the oil-soluble perfume is not contained, and it is particularly preferable that such solvent is not contained. Conversely, the water content in the solvent of the emulsion is preferably 40% by weight or more, more preferably 90% by weight or more, particularly preferably 100% by weight.

Other components which can be contained in the perfume layer may be suitably added in appropriate amounts to the emulsion, as described above.

After the emulsion is applied to the cleaning body, the solvent in the emulsion applied is removed in a drying step. The drying method may include, for example, an air drying method by ventilation, and the like. The ventilation temperature is appropriately determined within a range in which the oil-soluble perfume is not volatilized.

As a preferable embodiment of the method for manufacturing such interdental cleaning tool, for example, may be structured as follows. In a method for manufacturing an interdental cleaning tool comprising a handle base formed of a synthetic resin, and a cleaning part disposed at an end portion of the handle base, the method comprises a cleaning body-forming step in which the cleaning body, substantially formed of a polystyrene elastomer having a Shore hardness A of 30 to 60, more preferably 30 to 40, is provided at the end portion of the handle base; a coating step in which an emulsion containing, as a flavor component, at least one oil-soluble perfume selected from menthol and menthol analogs, as a water-soluble covering base, gum arabic and/or starch octenylsuccinate in a content of 5% by weight or more and 30% by weight or less, and as a solvent, water, is applied to a surface of the cleaning body; and a drying step in which the solvent is removed from the emulsion applied to the surface of the cleaning body.

Another preferable embodiment of the method for manufacturing such interdental cleaning tool, for example, may be structured as follows. In a method for manufacturing an interdental cleaning tool comprising a handle base formed of a synthetic resin and a cleaning part provided at an end portion of the handle base, the method comprises a cleaning body-forming step in which the cleaning body, substantially formed of a polystyrene elastomer having a Shore hardness A of 30 to 60, more preferably 30 to 40, is provided at the end portion of the handle base; a coating step in which an emulsion containing, as a flavor component, at least one oil-soluble perfume selected from menthol and menthol analogs, as a water-soluble covering base, gum arabic and/or starch octenylsuccinate in a content of 5% by weight or more and 30% by weight or less, and one or more surfactant selected from polyoxyethylene hardened castor oil, polyglycerin fatty acid ester, sorbitan fatty acid ester, and sucrose fatty acid ester in a content of less than 10% by weight, and as a solvent, water, is applied to a surface of the cleaning body; and a drying step in which the solvent is removed from the emulsion applied to the surface of the cleaning body.

In addition, another preferable embodiment of the method for manufacturing such an interdental cleaning tool, for example, may be structured as follows. In a method for manufacturing an interdental cleaning tool comprising a handle base formed of a synthetic resin and a cleaning part provided at an end portion of the handle base, the method comprises a cleaning body-forming step in which the cleaning body, substantially formed of a polystyrene elastomer having a Shore hardness A of 30 to 60, more preferably 30 to 40, is provided at the end portion of the handle base; a coating step in which an emulsion containing, as a flavor component, at least one oil-soluble perfume selected from menthol and menthol analogs, as a water-soluble covering base, gum arabic and/or starch octenylsuccinate in a content of 5% by weight or more and 30% by weight or less, and one or more surfactant selected from polyoxyethylene hardened castor oil, polyglycerin fatty acid ester, sorbitan fatty acid ester, and sucrose fatty acid ester in a content of less than 10% by weight, and as a solvent, water and an organic solvent in a solvent ratio of 30% by weight or less, is applied to a surface of the cleaning body; and a drying step in which the solvent is removed from the emulsion applied to the surface of the cleaning body.

### Example

The embodiments of the present invention are more specifically explained referring to Examples, but the present invention is not limited thereto.

### (Test Example 1)

A cleaning part was formed of a polystyrene elastomer at an end portion of a polypropylene handle base by injection molding, whereby an interdental cleaning tool having a structure as shown in Fig. 1 (a) was produced.

### (Test Example 2)

A cleaning part was formed of a polyester elastomer at an end portion of a polypropylene handle base by injection molding, whereby an interdental cleaning tool having a structure as shown in Fig. 1 (a) was produced.

### (Test Example 3)

A cleaning part was formed of a polyolefin elastomer at an end portion of a polypropylene handle base by injection molding, whereby an interdental cleaning tool having a structure as shown in Fig. 1 (a) was produced.

### (Evaluation test of sense of use)

20 interdental cleaning tools obtained in each of Test Examples 1, 2 and 3 were prepared, and 20 testers used them and evaluated, based on "favorable" or "unfavorable", the sense of touch when they cleaned between teeth using the tool.

**[Table 1]**

| | Kind of elastomer | Favorable | Unfavorable |
|---|---|---|---|
| Test Example 1 | Polystyrene elastomer | 20 | 0 |
| Test Example 2 | Polyester elastomer | 4 | 16 |
| Test Example 3 | Polyolefin elastomer | 10 | 10 |

As shown in Table 1, in the interdental cleaning tool in which the cleaning part was formed of the polystyrene elastomer, all of the testers stated that the tool had a favorable sense of use. From the above, it was confirmed that the polystyrene elastomer was a very appropriate material in terms of the gentle sense of touch as the elastomer used for the cleaning body of the interdental cleaning tool.

### (Example 1)

A polystyrene elastomer cleaning body was formed at an end portion of a polypropylene handle base by injection molding. In addition, an emulsion having a composition shown in Table 2 was prepared, and the emulsion was applied to the cleaning body by spraying and dried to obtain an interdental cleaning tool.

### (Example 2)

An interdental cleaning tool was obtained in the same manner as in Example 1 except that the composition of the emulsion was changed to that shown in Table 2.

### (Example 3)

An interdental cleaning tool was obtained in the same manner as in Example 1 except that the composition of the emulsion was changed to that shown in Table 2.

### (Example 4)

An interdental cleaning tool was obtained in the same manner as in Example 1 except that the composition of the emulsion was changed to that shown in Table 2.

### (Example 5)

An interdental cleaning tool was obtained in the same manner as in Example 1 except that the composition of the emulsion was changed to that shown in Table 2.

### (Example 6)

An interdental cleaning tool was obtained in the same manner as in Example 1 except that the composition of the emulsion was changed to that shown in Table 2.

### (Comparative Example 1)

An interdental cleaning tool was obtained in the same manner as in Example 1 except that the composition of the emulsion was changed to that shown in Table 2.

### (Comparative Example 2)

An interdental cleaning tool was obtained in the same manner as in Example 1 except that the composition of the emulsion was changed to that shown in Table 2.

### (Comparative Example 3)

An interdental cleaning tool was obtained in the same manner as in Example 1 except that the composition of the emulsion was changed to that shown in Table 2.

### (Comparative Example 4)

An interdental cleaning tool was obtained in the same manner as in Example 1 except that the composition of the emulsion was changed to that shown in Table 2.

### (Emulsion evaluation test)

The emulsions used in Examples 1 to 6 and Comparative Examples 1 to 4 were allowed to stand at room temperature in a dark place for one week, and the emulsification stability of the emulsion was evaluated by visually observing the separation state thereof. In addition, the surface of the cleaning part after the production in each Example and Comparative Example was visually observed, whereby the coatability thereof was evaluated. Furthermore, the product from each Example and Comparative Example was allowed to stand in an environment of room temperature, 45°C or 50°C for one to three month, and then the flavor of the cleaning part was evaluated, whereby the stability with time was evaluated.

**[Table 2]**

| | Example 1 | Example 2 | Example 3 | Example 4 | Example 5 | Example 6 | Comparative Example 1 | Comparative Example 2 | Comparative Example 3 | Comparative Example 4 |
|---|---|---|---|---|---|---|---|---|---|---|
| Peppermint | 2 | 2 | 2 | 2 | 2 | 2 | 2 | 2 | 2 | 2 |
| Menthol | 8 | 8 | 8 | 8 | 8 | 8 | 8 | 8 | 8 | 8 |
| Menthol derivative (*) | 1 | 1 | 1 | 1 | 1 | 1 | 1 | 1 | | 1 |
| Starch octenylsuccinate | | | | | | 15 | | 30 | | |
| Gum arabic | 10 | 15 | 15 | 20 | 30 | | | | | |
| Sucrose fatty acid ester | | | | | | | 5 | | | |
| Saccharin | 0.1 | 0.1 | 0.1 | 0.1 | 0.1 | 0.1 | 0.1 | 0.1 | | |
| Glycerin fatty acid ester | 2 | 2 | 2 | 2 | 2 | 2 | | | | |
| Sodium benzoate | 0.1 | 0.1 | | 0.1 | 0.1 | 0.1 | 0.1 | 0.1 | | |
| Ethyl paraben | | | 0.1 | | | | | | | |
| Citric acid | tr | tr | | tr | tr | tr | tr | tr | | |
| Sorbitol | | | | | | | | 10 | | |
| Ethanol | | | | | | | | | remainder | remainder |
| Water | remainder | remainder | remainder | remainder | remainder | remainder | remainder | remainder | | |
| Total | 100 | 100 | 100 | 100 | 100 | 100 | 100 | 100 | 100 | 100 |
| Emulsification (dissolution) stability | ○ | ○ | ○ | ○ | ○ | Δ | × | ○ | ○ | ○ |
| Coatablity | ○ | ○ | ○ | ○ | Δ | ○ | - | × | ○ | ○ |
| Stability with time | ○ | ○ | ○ | ○ | ○ | Δ | - | - | × | × |

| | | | | | | | | | | |
|---|---|---|---|---|---|---|---|---|---|---|
| <Emulsification (dissolution) stability> ○: No separation Δ: Slight separation ×: Complete separation <Coatablity> ○: Coatable ×: Impossible to coat -: Impossible to evaluate <Stability with time> ○: No problem in flavor Δ: Reduced flavor ×: No flavor -: Impossible to evaluate (*): As the menthol derivative, p-menthoxypropane-1,2-diol or 1-menthyl 3-hydroxybutyrate was used. tr: The component was contained, but the amount thereof did not reach the minimum description amount. | | | | | | | | | | |

As shown in Table 2, the results show that the interdental cleaning tools from Examples 1 to 6 in which the emulsion was prepared by containing the gum arabic and/or the starch octenylsuccinate were more excellent in the emulsification stability, the coatability, and the stability with time than those of the interdental cleaning tools from Comparative Examples. From the comparison of Examples 1 to 6 with Comparative Examples 3 and 4, it was confirmed that when the peppermint or menthol was prepared into the emulsion, the stability with time of the flavor component was more excellent compared to the case where they were simply coated on the cleaning body. When the gum arabic and/or the starch octenylsuccinate were not contained in the emulsion as in Comparative Example 1, or when too large amount of the starch octenylsuccinate was contained, it was confirmed that there were problems in terms of the emulsification stability or the coatability.

### (Test Example 4)

A flat plate-shaped polystyrene elastomer test piece having a Shore hardness A of 30 was formed.

### (Test Example 5)

A flat plate-shaped polystyrene elastomer test piece having a Shore hardness A of 40 was formed.

### (Test Example 6)

A flat plate-shaped polystyrene elastomer test piece having a Shore hardness A of 50 was formed.

### (Test Example 7)

A flat plate-shaped polystyrene elastomer test piece having a Shore hardness A of 60 was formed.

### (Test Example 8)

A flat plate-shaped polyethylene terephthalate (hereinafter referred to as PET) rigid resin test piece was formed.

### (Test Example 9)

A flat plate-shaped low density polyethylene (hereinafter referred to as LDPE) rigid resin test piece was formed.

### (Test Example 10)

A flat plate-shaped polyolefin (hereinafter referred to as EPDM) elastomer test piece was formed.

### (Test Example 11)

A flat plate-shaped polyester elastomer test piece was formed.

### (Liquid dripping evaluation test)

An emulsion having the same composition as that of the emulsion used in the production of the interdental cleaning tool in Examples 1, 2, 4 and 5 was added dropwise, by using a 0.25 mL syringe, to the flat plate-shaped test piece from each Test Example 4 to 11 in a state in which the piece was tilted by an angle of 20°, 30°, or 40° from the horizontal state and whether or not liquid droplets applied to the test piece easily cause the liquid dripping was evaluated.

**[Table 3]**

| | Material forming cleaning body | | Shore hardness A | Tilt 20° | | | |
|---|---|---|---|---|---|---|---|
| | | | | Example 1 | Example 2 | Example 4 | Example 5 |
| Test Example 4 | Elastomer | Polystyrene | 30 | ⊚ | ⊚ | ⊚ | ⊚ |
| Test Example 5 | Elastomer | Polystyrene | 40 | ⊚ | ⊚ | ⊚ | ⊚ |
| Test Example 6 | Elastomer | Polystyrene | 50 | ⊚ | ⊚ | ⊚ | ⊚ |
| Test Example 7 | Elastomer | Polystyrene | 60 | ⊚ | ⊚ | ⊚ | ⊚ |
| Test Example 8 | Rigid resin | PET | - | ○ | ○ | ○ | × |
| Test Example 9 | Rigid resin | LDPE | - | × | × | × | × |
| Test Example 10 | Elastomer | EPDM | - | ⊚ | ⊚ | ⊚ | ⊚ |
| Test Example 11 | Elastomer | Polyester | - | ⊚ | ⊚ | ⊚ | ⊚ |

| | Material forming cleaning body | | Shore hardness A | Tilt 30° | | | |
|---|---|---|---|---|---|---|---|
| | | | | Example 1 | Example 2 | Example 4 | Example 5 |
| Test Example 4 | Elastomer | Polystyrene | 30 | ⊚ | ⊚ | ⊚ | ○ |
| Test Example 5 | Elastomer | Polystyrene | 40 | ⊚ | ⊚ | ⊚ | ○ |
| Test Example 6 | Elastomer | Polystyrene | 50 | ⊚ | ⊚ | ⊚ | ○ |
| Test Example 7 | Elastomer | Polystyrene | 60 | ⊚ | ⊚ | ⊚ | ○ |
| Test Example 8 | Rigid resin | PET | - | × | × | × | × |
| Test Example 9 | Rigid resin | LDPE | - | × | × | × | × |
| Test Example 10 | Elastomer | EPDM | - | × | × | × | × |
| Test Example 11 | Elastomer | Polyester | - | ○ | ○ | ○ | ○ |

| | Material forming cleaning body | | Shore hardness A | Tilt 40° | | | |
|---|---|---|---|---|---|---|---|
| | | | | Example 1 | Example 2 | Example 4 | Example 5 |
| Test Example 4 | Elastomer | Polystyrene | 30 | ○ | ○ | ○ | ○ |
| Test Example 5 | Elastomer | Polystyrene | 40 | ○ | ○ | ○ | ○ |
| Test Example 6 | Elastomer | Polystyrene | 50 | ○ | ○ | ○ | ○ |
| Test Example 7 | Elastomer | Polystyrene | 60 | ○ | ○ | ○ | ○ |
| Test Example 8 | Rigid resin | PET | - | × | × | × | × |
| Test Example 9 | Rigid resin | LDPE | - | × | × | × | × |
| Test Example 10 | Elastomer | EPDM | - | × | × | × | × |
| Test Example 11 | Elastomer | Polyester | - | × | × | × | × |

| | | | | | | | |
|---|---|---|---|---|---|---|---|
| ⊚: No dripping ○: Dripping up to 5 mm ×: Dripping 5 mm or more | | | | | | | |

As shown in Table 3, it was suggested that in the test pieces from Test Examples 4 to 7 produced from the polystyrene elastomer having a Shore hardness A of 30 to 60, the liquid dripping was remarkably suppressed compared to the other test pieces from Test Examples 8 to 11. From the results described above, it was suggested that when the cleaning body was formed of the polystyrene elastomer having a Shore hardness A of 30 to 60, a larger amount of the emulsion could be applied and remained on the cleaning body when it was coated. It was suggested, accordingly, that when the cleaning body was formed of the polystyrene elastomer having a Shore hardness A of 30 to 60, and the resulting cleaning body was coated with the emulsion having the flavor component, the cool sensation caused by the flavor component could be maintained for a longer period of time.

### Reference Signs List

- 1: Interdental cleaning tool
- 2: Handle base
- 3: Cleaning part
- 4: Cleaning body
- 5: Protrusion

## Claims

1. An interdental cleaning tool comprising: a handle base formed of a synthetic resin; and a cleaning part disposed on an end portion of the handle base, wherein
a surface of a cleaning body substantially formed of a polystyrene elastomer, characterised therein that the cleaning part is covered with a perfume layer, and the perfume layer contains a glycerine fatty acid ester, an oil-soluble perfume as a flavor component, and gum arabic and/or starch octenylsuccinate as a water-soluble covering base.

2. The interdental cleaning tool according to claim 1, wherein the polystyrene elastomer has a Shore hardness A of 30 to 60.

3. The interdental cleaning tool according to claim 1 or 2, wherein the oil-soluble perfume contains at least one member selected from menthol and menthol analogues.

4. The interdental cleaning tool according to any of claims 1 to 3, wherein the perfume layer contains the gum arabic and/or the starch octenylsuccinate in a content of 40% by weight or more and 70% by weight or less.

5. The interdental cleaning tool according to any of claims 1 to 4, wherein the cleaning body is formed so as to cover a surface of the end portion of the handle base.

6. The interdental cleaning tool according to any of claims 1 to 5, wherein the cleaning body comprises a protrusion radially formed from the handle base.

7. A method for manufacturing an interdental cleaning tool comprising a handle base formed of a synthetic resin, and a cleaning part disposed on an end portion of the handle base, comprising:
a cleaning body-forming step in which a cleaning body substantially formed of a polystyrene elastomer is disposed on the end portion of the handle base;
**characterised by** a coating step in which a surface of the cleaning body is coated with an emulsion containing a glycerine fatty acid ester, an oil-soluble perfume as a flavor component, gum arabic and/or starch octenylsuccinate as a water-soluble covering base, and water as a solvent; and
a drying step in which the solvent is removed from the emulsion coated on the surface of the cleaning body.

8. The method for manufacturing an interdental cleaning tool according to claim 7, wherein the emulsion contains the gum arabic and/or the starch octenylsuccinate in a content of 5% by weight or more and 30% by weight or less.

9. The method for manufacturing an interdental cleaning tool according to claim 7 or 8, wherein, in the coating step, the surface of the cleaning body is spray-coated with the emulsion.

10. The method for manufacturing an interdental cleaning tool according to any of claims 7 to 9, wherein the water in the solvent is contained in a content of 40% by weight or more.

## Patentansprüche

1. Interdentales Reinigungsinstrument, das aufweist: eine aus einem Kunstharz gebildete Griffbasis; und eine Reinigungskomponente, die an einem Endbereich der Griffbasis angeordnet ist, wobei eine Oberfläche eines Reinigungskörpers im Wesentlichen aus einem Polystyrol-Elastomer ausgebildet ist, **dadurch gekennzeichnet, dass** die Reinigungskomponente mit einer Parfümschicht bedeckt ist, und
die Parfümschicht einen Glycerinfettsäureester, ein öllösliches Parfüm als Geschmackskomponente und Gummiarabikum und/oder Stärke-Octenylsuccinat als wasserlösliche Überzugsbasis aufweist.

2. Interdentales Reinigungsinstrument nach Anspruch 1, wobei das Polystyrol-Elastomer eine Shore-Härte A von 30 bis 60 hat.

3. Interdentales Reinigungsinstrument nach Anspruch 1 oder 2, wobei das öllösliche Parfüm wenigstens ein Element aufweist, das ausgewählt ist aus Menthol und Mentholanaloga.

4. Interdentales Reinigungsinstrument nach einem der Ansprüche 1 bis 3, wobei die Parfümschicht das Gummiarabikum und/oder das Stärke-Octenylsuccinat in einem Gewichtsanteil von 40 % oder mehr bis zu einem Gewichtsanteil von 70 % oder weniger aufweist.

5. Interdentales Reinigungsinstrument nach einem der Ansprüche 1 bis 4, wobei der Reinigungskörper ausgebildet ist, um eine Oberfläche des Endbereichs der Griffbasis abzudecken.

6. Interdentales Reinigungsinstrument nach einem der Ansprüche 1 bis 5, wobei der Reinigungskörper einen Vorsprung aufweist, der radial aus der Griffbasis ausgebildet ist.

7. Verfahren zum Herstellen eines interdentalen Reinigungsinstruments, das aufweist eine aus einem Kunstharz gebildete Griffbasis und eine Reinigungskomponente, die an einem Endbereich der Griffbasis angeordnet ist, das aufweist:
einen Ausbildungschritt des Reinigungskörpers, in dem ein im Wesentlichen aus einem Polystyrol-Elastomer gebildeter Reinigungskörper am Endbereich der Griffbasis angeordnet wird,
**gekennzeichnet durch**
einen Beschichtungsschritt, in dem eine Oberfläche des Reinigungskörpers mit einer Emulsion beschichtet wird, die einen Glycerinfettsäureester, ein öllösliches Parfüm als eine Geschmackskomponente, Gummiarabikum und/oder Stärke-Octenylsuccinat als wasserlösliche Beschichtungsbasis und Wasser als Lösungsmittel aufweist;
und einen Trocknungsschritt, in dem das Lösungsmittel aus der auf der Oberfläche des Reinigungskörpers beschichteten Emulsion entfernt wird.

8. Verfahren zum Herstellen eines interdentalen Reinigungsinstruments nach Anspruch 7, wobei die Emulsion das Gummiarabikum und/oder das Stärke-Octenylsuccinat in einem Gewichtsanteil von 5% oder mehr bis zu einem Gewichtsanteil von 30% oder weniger aufweist.

9. Verfahren zum Herstellen eines interdentalen Reinigungsinstruments nach Anspruch 7 oder 8, wobei im Beschichtungsschritt die Oberfläche des Reinigungskörpers durch Sprühen mit der Emulsion beschichtet wird.

10. Verfahren zum Herstellen eines interdentalen Reinigungsinstruments nach einem der Ansprüche 7 bis 9, wobei das Wasser im Lösungsmittel in einem Gewichtsanteil von 40 % oder mehr enthalten ist.

## Revendications

1. Outil de nettoyage interdentaire comprenant : une base de manche formée d'une résine synthétique ; une partie de nettoyage disposée sur une portion d'extrémité de la base de manche, dans lequel une surface d'un corps de nettoyage substantiellement formé d'un élastomère de polystyrène, **caractérisé en ce que**
la partie de nettoyage est couverte avec une couche de parfum, et la couche de parfum contient un ester d'acide gras de glycérine, un parfum soluble dans l'huile comme un constituant d'arôme, et de la gomme arabique et/ou de l'octénylsuccinate d'amidon comme base de couverture soluble dans l'eau.

2. Outil de nettoyage interdentaire selon la revendication 1, dans lequel l'élastomère de polystyrène présente une dureté Shore A de 30 à 60.

3. Outil de nettoyage interdentaire selon la revendication 1 ou 2, dans lequel le parfum soluble dans l'huile contient au moins un élément choisi parmi le menthol et des analogues de menthol.

4. Outil de nettoyage interdentaire selon l'une quelconque des revendications 1 à 3, dans lequel la couche de parfum contient la gomme arabique et/ou l'octénylsuccinate d'amidon dans une teneur de 40 % en masse ou supérieure et de 70 % en masse ou inférieure.

5. Outil de nettoyage interdentaire selon l'une quelconque des revendications 1 à 4, dans lequel le corps de nettoyage est formé afin de couvrir une surface de la portion d'extrémité de la base de manche.

6. Outil de nettoyage interdentaire selon l'une quelconque des revendications 1 à 5, dans lequel le corps de nettoyage comprend une protubérance radialement formée à partir de la base de manche.

7. Procédé de fabrication d'un outil de nettoyage interdentaire comprenant une base de manche formée d'une résine synthétique, et une partie de nettoyage disposée sur une portion d'extrémité de la base de manche, comprenant :
une étape de formation de corps de nettoyage dans laquelle un corps de nettoyage substantiellement formé d'un élastomère de polystyrène est disposé sur la portion d'extrémité de la base de manche ;
**caractérisé par**
une étape de revêtement dans laquelle une surface du corps de nettoyage est revêtue avec une émulsion contenant un ester d'acide gras de glycérine, un parfum soluble dans l'huile comme un constituant d'arôme, de la gomme arabique et/ou de l'octénylsuccinate d'amidon comme une base de couverture soluble dans l'eau, et de l'eau comme un solvant ; et
une étape de séchage dans laquelle le solvant est éliminé de l'émulsion déposée sur la surface du corps de nettoyage.

8. Procédé de fabrication d'un outil de nettoyage interdentaire selon la revendication 7, dans lequel l'émulsion contient la gomme arabique et/ou l'octénylsuccinate d'amidon dans une teneur de 5 % en masse ou supérieure et de 30 % en masse ou inférieure.

9. Procédé de fabrication d'un outil de nettoyage interdentaire selon la revendication 7 ou 8, dans lequel, dans l'étape de revêtement, la surface du corps de nettoyage est revêtue par pulvérisation avec l'émulsion.

10. Procédé de fabrication d'un outil de nettoyage interdentaire selon l'une quelconque des revendications 7 à 9, dans lequel l'eau dans le solvant est contenue dans une teneur de 40 % en masse ou supérieure.
